# EUROPEAN PATENT SPECIFICATION

(11) **EP 1 222 200 B1**
(45) Date of publication and mention of the grant of the patent: **21.12.2005**
(21) Application number: 00966633.0
(22) Date of filing: 19.09.2000
(51) Int. Cl.: C07K 14/025, C12N 15/86, A61K 48/00

(54) **Fusion protein between HPV-L1 and a peptide, capable of forming a VLP and that functions as a vehicle for introduction of a peptide in a cell and medical uses thereof**
Fusionsprotein aus HPV-L1 und einem Peptid, das ein VLP bilden kann und als Vehikel zum EInschleusen eines Peptids in eine Zelle dienen kann, sowie dessen medizinische Verwendungen
Protéine de fusion entre HPV-L1 et un peptide, capable de former un VLP et fonctionnant comme véhicule pour l'introduction d'un peptide dans une cellule, et ses utilisations médicales

(30) Priority: 30.09.1999 SE 9903534
(43) Date of publication of application: 17.07.2002
(73) Proprietor: Active Biotech AB, 220 07 Lund (SE)
(72) Inventor: ANTONSSON, Per, S-226 52 Lund (SE); KRISTENSSON, Karin, S-222 20 Lund (SE); WALLEN-ÖHMAN, Marie, S-227 30 Lund (SE); DILLNER, Joakim, S-182 35 Danderyd (SE); LANDO, Peter, S-211 46 Malmö (SE)
(74) Representative: Stenbäck, Maria Elisabeth
(86) International application number: PCT/SE2000/001808
(87) International publication number: WO 2001/023422

(56) References cited:
- WO-A1-99/15630
- WO-A2-96/11272
- WO-A2-99/48518
- DATABASE MEDLINE [Online] NATIONAL LIBRARY OF MEDICINE HINES J.F. ET AL.: 'The expressed L1 proteins of HPV-1, HPV-6 and HPV-11 display type specific epitopes with native conformation and reactivity with neutralizing and nonneutralizing antibodies', XP002935318 Database accession no. 95251779 & PATHOBIOLOGY vol. 62, no. 4, 1994, pages 165 - 171

## Description

### FIELD OF THE INVENTION

The present invention relates to a carrier for introduction of substances into cells comprising a modified major capsid protein L1 of human papillomavirus (HPV-L1 protein) devoid of type-specific epitopes causing production of neutralising antibodies. The invention also includes an oligo- or polynucleotide coding for said carrier, vaccines comprising said carrier or said oligo- or polynucleotide, as well as methods of using the carrier or the oligo- or polynucleotide in vaccination against viral, bacterial or parasite infections as well as against development of certain cancers. Especially, infections of human papillomavirus and the development of cancer as a consequence of such infections are recognised.

### BACKGROUND OF THE INVENTION

The Human Papillomavirus (HPV) is since long established as the major cause of cervical cancer (1), and has in recent years also been established as a cause of cancers of the penis, vulva, vagina, anus and orofarynx. There also exists indications that the virus may be involved in some cancers of the prostate, esophagus and in other head and neck cancers. HPV vaccine development is therefore a prime priority of preventive cancer research today (2).

The HPVs exist as >100 different types. Although types are defined by genetic homology, the genotypes have hitherto shown a strikingly good concordance with sero-types, i.e. hyperimmune antisera against one type will only neutralise the same type and not other genotypes. Cross-neutralisations have only been reported for certain closely related types and have had titers 2 orders of magnitude less than for the type-specific neutralisation (2,3).

The HPV capsid consists of 72 capsomers each containing 5 copies of the HPV major capsid protein L1. A minor capsid protein, L2, is present in much smaller amounts in the capsid (1:12 compared to the L1 protein) and the location of L2 is uncertain (2).

A number of small viruses express capsid proteins that when expressed self-assemble to form virus-like particles (VLPs) (i.e. particles morphologically similar to virus particles, but lacking the viral genome). The HPV major capsid protein L1 is among the best studied (2). HPV VLPs containing only L1 are morphologically similar to VLPs containing both L1 and L2 (2). Both particles with L1 only and particles with L1/L2 are highly efficient in eliciting a high-titered neutralising antibody response in several animal model systems (rabbits, cows, dogs and rhesus monkeys), even when injected in the absence of adjuvant (2).

Vaccination with papillomavirus VLPs has been shown to be highly efficient for protection, mediated by neutralising antibodies, against subsequent challenge with both cutaneous and mucosal papillomaviruses, but only in a type-specific manner (2). This strong type-specificity is surprising, since the major capsid protein of the HPVs is a highly evolutionarily conserved protein with very few amino acid changes between genetically related, but not cross-neutralising, HPV types.

The most common oncogenic HPVs are HPV16, 18, 31 and 45. HPV16 is found in about 50% of cervical cancers, HPV18 in about 20%, and these four types together correspond to >80% of all cervical cancers. Therefore, a commonly contemplated strategy is to manufacture vaccines containing HPV capsids of the 4 most common HPV types together (2).

Albeit this strategy appears likely to work for achieving significant cancer reduction, it has some distinct disadvantages. The formulation of vaccines containing 4 active components mixed together involves a substantial additional cost in manufacturing and efficacy testing and quality control of each component.

Furthermore, some 10-20% of cervical cancers are caused by HPV types not included in the presently manufactured vaccine candidates. Apart from the fact that the vaccine could not possibly protect against these types, the possibility also exists that elimination of the 4 most common oncogenic HPV types may cause an increase in the prevalence of the other oncogenic HPV types, thus further diminishing the cancer-preventive gains. This latter scenario is, as predicted from population biology studies, likely to follow if there exists interference between different viral types. Several lines of indirect evidence do indicate that interference between HPV types does exist.

Several other HPV types cause significant morbidity and mortality, most notably HPV 6 and 11 that cause genital condylomas and recurrent respiratory papillomatoses, and HPVs 5 and 8 that cause cutaneous skin-cancers in the immunosuppressed host.In spite of the obvious advantages of broadly cross-reactive vaccines, the possibility to generate a broadly cross-reactive vaccine, by modifying the L1 protein to not contain immunodominant type-specific epitopes, has not been proposed. Several surface exposed and cross-reactive epitopes are exposed on papillomavirus particles (WO 96/33737), but are not immunogenic in the presence of the immunodominant type-specific epitope (4). Therefore, by modifying the L1 to remove immunodominant type-specific epitopes, it should be possible to generate a cross-reactive papillomavirus vaccine, using a modified HPV-L1 protein as a carrier of surface exposed HPV derived antibody epitopes.

Furthermore, VLPs are highly efficient in eliciting a cytotoxic T lymphocyte (CTL) response, and VLP vaccines have been reported to be highly efficacious (through a CD8+cell-dependent mechanism) in preventing and treating transplantable cancers in several mouse models, in spite of the fact that immunization is made with an exogenous protein (5). The high immunogenicity appears to be due in part to the preservation of an active mechanism for infection of the cell (designated pseudo-infection, as no viral genome is introduced) which results in the capsid protein being processed and presented in the MHC class I presentation pathway (6). VLPs are therefore of general interest from a vaccine biotechnology point of view, since they can be used as a vehicle for efficient immunogenic delivery of any antigen (7).

Efficient immunisation using wild-type HPV VLPs carrying foreign antigens has been demonstrated in several systems, e.g. the MAGE melanoma antigens and human immunodeficiency virus antigens.

A potential problem using VPLs as vehicles for immunogenic delivery is blocking by type-specific neutralising antibodies. In Sweden 16% of the adult population are sero-positive for HPV-16, reflecting the importance of the problem. In addition, therapeutic vaccination is expected to require recurrent treatments, likely to induce a type-specific antibody response towards a wild-type VLP carrier.

Therefore, by modifying the L1 protein to remove type-specific epitopes causing production of neutralising antibodies, as has been described (8), and introduce antibody or T-cell epitopes in this carrier, it should be possible to generate an immunological response towards the introduced peptide, without obstruction from type-specific neutralising antibodies directed towards the carrier itself.

### SUMMARY OF THE INVENTION

An object of the present invention is to provide means for preventing and treating viral, bacterial or parasite infections, especially of human papilloma virus, and the development of benign or malign consequences of such infections, as well as means for treating and preventing cancer.

The present invention provides for the use of a modified HPV-L1 protein devoid of type-specific epitopes causing production of neutralising antibodies, as a carrier of a substance into cells. As a result of the modification, this HPV-L1 protein carrier does not induce production of overt neutralising antibodies towards the carrier itself. In an embodiment of the invention, one or more amino acids may be deleted from said protein.

In particular, the invention provides for such an HPV-L1 protein in fusion with a peptide.

The invention also provides for such a carrier which is capable of giving rise to a protective antibody response, which antibody response may be cross-reactive towards two or more serologically defined subtypes of human papillomavirus.

The carrier must be physically coupled, that is fused, to the peptide for which it acts as a carrier, thus creating a fusion protein.

Particularly, peptides derived from HPV proteins and defining linear antibody epitopes and T-cell epitopes are recognised.

There is also envisaged combinations of said carrier with a minor coat protein of human papillomavirus (HPV-L2 protein), native or modified. Also this HPV-L2 protein can itself be fused to one or more further peptides.

The invention also provides for an oligo- or polynucleotide coding for said carrier. The invention makes it possible to create a better basis for eliciting an MHC class I mediated response, i.e. creating cytotoxic T-cells, without giving rise to type-specific neutralising antibodies towards the carrier, or without type-specific neutralising antibodies being present at the start.

It is also possible to use an HPV-L1 protein, modified as described above, as a carrier of oligo- or polynucleotides to cells.

### DETAILED DESCRIPTION OF THE INVENTION

In one of its aspects, the invention provides for a carrier in the form of a fusion protein capable of forming a VLP which functions as a vehicle for introduction of a peptide into cells, comprising a major capsid protein L1 of human papillomavirus (HPV-L1 protein) which has been intentionally modified to remove major type-specific epitope(s) causing production of neutralising antibodies, wherein said HPV-L1 protein is in fusion with the peptide.

Preferably, said peptide comprises one or more T-cell epitopes, especially such epitopes derived from tumor, bacterial, parasite, viral or auto-antigens. In another preferred embodiment, said peptide comprises one or more antibody epitopes, such as tumor, bacterial, parasite, viral or auto-antigens, especially papillomavirus antigens.

The carrier can also be combined with a minor capsid protein L2 of human papillomavirus (HPV-L2 protein), which in its turn may be fused to one or more further peptides. These further peptides are e.g. T-cell or antibody epitopes, which may be derived from tumor, bacterial, parasite, viral or auto-antigens.

In a further embodiment the fusion protein is used as a carrier of oligo- or polynucleotides, e.g. such oligo- or polynucleotides which are coding for an antigen or an immunostimulatory (poly)peptide.

In another aspect, the invention provides for an oligo- or polynucleotide coding for the carrier as defined.

In further aspects, the invention provides for vaccines, comprising as an active ingredient a carrier or an oligo- or polynucleotide as defined above.

In further aspects of the invention there is provided methods of preventing or treating viral, bacterial or parasite infections by vaccination with a carrier or an oligo- or polynucleotide as defined above. In a preferred embodiment the infections is caused by papillomavirus.

There is also provided methods of preventing or treating development of benign or malign consequences of human papillomavirus infection by vaccination with a fusion protein or an oligo- or polynucleotide as defined above.

In embodiments of the methods said human papillomavirus infection is warts or laryngeal papillomatosis.

Further aspects of the invention comprise methods of preventing or treating of cancer, including cancer of cervix, penis, vulva, vagina, anus and orofarynx, by vaccination with a fusion protein or an oligo- or polynucleotide as defined above.

### REFERENCES

- 1. H. zur Hausen. Viruses in human cancers. Science 1991; 254: 1167-1173.
- 2. D. R. Lowy and J. T. Schiller. Papillomaviruses and cervical cancer: Pathogenesis and vaccine development. J. Natl. Cancer Inst. Monographs 1998; 23: 27-30.
- 3. W. I. White, S. D. Wilson, W. Bonnez, R. C. Rose, S. Koenig and J. A. Suzich. In vitro infection and type-restricted antibody-mediated neutralization of authentic human papillomavirus type 16. J. Virol. 1998; 72: 959-964.
- 4. H. L. Greenstone, J. D. Nieland, K. E. deVisser, M. E. De Bruijn, R. Kirnbauer, R. B. Roden, D. R. Lowy, W. M. Kast and J. T. Schiller. Chimeric papillomavirus virus-like particles elicit antitumor immunity against the E7 oncoprotein in an HPV16 tumor model. Proc. Natl. Acad. Sci. USA 1998; 95: 1800-1805.
- 5. S. Peng, I. H. Frazer, G. J. Fernando and J. Zhou. Papillomavirus virus-like particles can deliver defined CTL epitopes to the MHC class I pathway. Virology 1998; 240: 147-157.
- 6. M. Muller, J. Zhou, T. D. Reed, C. Rittmuller, A. Burger, J. Gabelsberger, J. Braspenning and L. Gissmann. Chimeric papillomavirus-like particles. Virology 1997; 234: 93-111.
- 7. White, W.I., Wilson, S.D., Palmer-Hill, F.J., Woods, R.M., Ghim, S.-J., Hewitt, L.A., Goldman, D.M., Burke, S.J., Jenson, A.B., Koenig, S. and Suzich, J.A.: Characterization of a Major Neutralizing Epitope on Human Papillomavirus Type 16 L1. Virology, 1999; 73:4882-4889.
- 8. Wang, Z., Christensen, N.D., Schiller, J.T. and Dillner, J.: A monoclonal antibody against intact Human Papillomavirus type 16 capsids blocks the serological reactivity of most human sera. J. Gen. Virol., 78, 2209-2215 (1997).

## Claims

1. A fusion protein capable of forming a VLP which functions as a vehicle for introduction of a peptide into cells, comprising a major capsid protein L1 of human papillomavirus (HPV-L1 protein) which has been intentionally modified to remove major type-specific epitope(s) causing production of neutralising antibodies, wherein said HPV-L1 protein is in fusion with the peptide.

2. A fusion protein according to claim 1, wherein one or more amino acids have been deleted.

3. A fusion protein according to claim 1, wherein said peptide comprises one or more T-cell epitopes.

4. A fusion protein according to claim 3, wherein said one or more T-cell epitopes are derived from a group of antigens comprising tumor, bacterial, parasite, viral or auto-antigens.

5. A fusion protein according to claim 1, wherein said peptide comprises one or more antibody epitopes.

6. A fusion protein according to claim 5, wherein said one or more antibody epitopes are derived from a group of antigens comprising tumor, bacterial, parasite, viral or auto-antigens.

7. A fusion protein according to claim 6, wherein said one or more antibody epitopes are derived from human papillomavirus antigens.

8. A fusion protein according any one of claims 5-7, capable of giving rise to a protective antibody response.

9. A fusion protein according to claim 8, wherein said protective antibody response is cross-reactive towards two or more serologically defined subtypes of human papillomaviruses.

10. A fusion protein according to claim 9, wherein said protective respones is raised against two or more of the group comprising HPV-L1 proteins derived from human papillomavirus implicated in tumor induction.

11. A fusion protein according to claim 10, wherein said protective antibody response is cross-reactive towards two or more of the group of HPV-L1 proteins comprising L1 proteins of HPV-16, HPV-18, HPV-31 and HPV-45.

12. A fusion protein according to any one of claims 1-11 in combination with a minor capsid protein L2 of human papillomavirus (HPV-L2 protein).

13. A fusion protein according to claim 12, wherein said HPV-L2 protein is in fusion with one or more further peptides.

14. A fusion protein according to claim 13, wherein said one or more further peptides are chosen from a group of antigens comprising tumor, bacterial, parasite, viral and auto-antigens.

15. A vaccine, comprising as an active ingredient a fusion protein as defined in any one of claims 1-14.

16. A polynucleotide coding for the fusion protein as defined in any one of claims 1-14.

17. A vaccine, comprising as an active ingredient a polynucleotide as defined in claim 16.

18. A fusion protein as defined in any one of claims 1-14 for use as a medicament.

19. Use of a fusion protein according to any one of claims 1-14 for the manufacture of a vaccine for preventing or treating viral, bacterial or parasite infections.

20. Use according claim 19, for preventing or treating infection of human papillomavirus.

21. Use of a fusion protein according to any one of claims 1-14 for the manufacture of a vaccine for preventing or treating development of benign or malign consequences of human papillomavirus infection.

22. Use according to claim 21, whereby said human papillomavirus infection is chosen from the group comprising warts and laryngeal papillomatosis.

23. Use of a fusion protein according to any one of claims 1-14 for the manufacture of a vaccine for preventing or treating cancer.

24. Use according to claim 23, whereby said cancer is chosen from the group comprising cancer of cervix, penis, vulva, vagina, anus and orofarynx.

## Patentansprüche

1. Fusionsprotein, das in der Lage ist, ein VLP zu bilden, das als Vehikel zum Einführen eines Peptids in Zellen fungiert, umfassend ein Haupt-Kapsidprotein L1 des humanen Papillomavirus (HPV-L1-Protein), das absichtlich so modifiziert wurde, dass ein typspezifisches Haupt-Epitop (typspezifische Haupt-Epitope) entfernt wurde(n), welches (welche) die Produktion von neutralisierenden Antikörpern bewirkt (bewirken), wobei das HPV-L1-Protein in Fusion mit dem Peptid vorliegt.

2. Fusionsprotein nach Anspruch 1, wobei eine oder mehrere Aminosäuren deletiert wurden.

3. Fusionsprotein nach Anspruch 1, wobei das Peptid ein oder mehrere T-Zell-Epitope umfasst.

4. Fusionsprotein nach Anspruch 3, wobei das eine oder die mehreren T-Zell-Epitope von einer Gruppe von Antigenen stammen, die Tumor-, Bakterien-, Parasiten-, Virus- oder Auto-Antigene umfasst.

5. Fusionsprotein nach Anspruch 1, wobei das Peptid ein oder mehrere Antikörper-Epitope umfasst.

6. Fusionsprotein nach Anspruch 5, wobei das eine oder die mehreren Antikörper-Epitope aus einer Gruppe von Antigenen stammen, die Tumor-, Bakterien-, Parasiten-, Virus- oder Auto-Antigene umfasst.

7. Fusionsprotein nach Anspruch 6, wobei das eine oder die mehreren Antikörper-Epitope von Antigenen des humanen Papillomavirus stammen.

8. Fusionsprotein nach einem der Ansprüche 5 bis 7, das in der Lage ist, eine schützende Antikörperantwort hervorzubringen.

9. Fusionsprotein nach Anspruch 8, wobei die schützende Antikörperantwort gegenüber zwei oder mehr serologisch definierten Subtypen von humanen Papillomaviren kreuzreaktiv ist.

10. Fusionsprotein nach Anspruch 9, wobei die schützende Antwort gegen zwei oder mehrere Vertreter der Gruppe hervorgebracht wird, die HPV-L1-Proteine umfasst, die von einem humanen Papillomavirus stammen, das an der Induktion von Tumoren beteiligt ist.

11. Fusionsprotein nach Anspruch 10, wobei die schützende Antikörperantwort gegenüber zwei oder mehreren Vertretern der Gruppe von HPV-L1-Proteinen, die L1-Proteine von HPV-16, HPV-18, HPV-31 und HPV-45 umfasst, kreuzreaktiv ist.

12. Fusionsprotein nach einem der Ansprüche 1 bis 11 in Kombination mit einem Neben-Kapsidprotein L2 des humanen Papillomavirus (HPV-L2-Protein).

13. Fusionsprotein nach Anspruch 12, wobei das HPV-L2-Protein in Fusion mit einem oder mehreren weiteren Peptiden vorliegt.

14. Fusionsprotein nach Anspruch 13, wobei das eine oder die mehreren weiteren Peptide aus einer Gruppe von Antigenen ausgewählt sind, die Tumor-, Bakterien-, Parasiten-, Virus- und Auto-Antigene umfasst.

15. Impfstoff, der als einen Wirkstoff ein Fusionsprotein gemäß der Definition in einem der Ansprüche 1 bis 14 umfasst.

16. Polynucleotid, welches das Fusionsprotein gemäß der Definition in einem der Ansprüche 1 bis 14 codiert.

17. Impfstoff, der als einen Wirkstoff ein Polynucleotid gemäß der Definition in Anspruch 16 umfasst.

18. Fusionsprotein gemäß der Definition in einem der Ansprüche 1 bis 14 zur Verwendung als Medikament.

19. Verwendung eines Fusionsproteins nach einem der Ansprüche 1 bis 14 zum Herstellen eines Impfstoffs zum Verhindern oder Behandeln von Virus-, Bakterien- oder Parasiten-Infektionen.

20. Verwendung nach Anspruch 19 zum Verhindern oder Behandeln einer Infektion mit einem humanen Papillomavirus.

21. Verwendung eines Fusionsproteins nach einem der Ansprüche 1 bis 14 zum Herstellen eines Impfstoffs zum Verhindern oder Behandeln der Entwicklung von benignen oder malignen Folgen einer Infektion mit einem humanen Papillomavirus.

22. Verwendung nach Anspruch 21, wobei die Infektion mit einem humanen Papillomavirus aus der Gruppe ausgewählt ist, die Warzen und die Larynx-Papillomatose umfasst.

23. Verwendung eines Fusionsproteins nach einem der Ansprüche 1 bis 14 zum Herstellen eines Impfstoffs zum Verhindern oder Behandeln von Krebs.

24. Verwendung nach Anspruch 23, wobei der Krebs aus der Gruppe ausgewählt ist, die Krebs der Zervix, des Penis, der Vulva, der Vagina, des Anus und des Oropharynx umfasst.

## Revendications

1. Protéine de fusion capable de former une VLP qui fonctionne comme un véhicule pour l'introduction d'un peptide dans des cellules, comprenant une protéine de capside majeure L1 de papillomavirus humain (protéine HPV-L1) qui a été modifiée volontairement pour retirer le ou les épitopes spécifiques de types majeurs causant la production d'anticorps neutralisants, où ladite protéine HPV-L1 est fusionnée avec le peptide.

2. Protéine de fusion selon la revendication 1 où un ou plusieurs aminoacides ont été délétés.

3. Protéine de fusion selon la revendication 1 où ledit peptide comprend un ou plusieurs épitopes de cellules T.

4. Protéine de fusion selon la revendication 3 où ledit ou lesdits épitopes de cellules T sont dérivés d'un groupe d'antigènes comprenant des antigènes tumoraux, bactériens, parasitaires, viraux ou des auto-antigènes.

5. Protéine de fusion selon la revendication 1 où ledit peptide comprend un ou plusieurs épitopes d'anticorps.

6. Protéine de fusion selon la revendication 5 où le ou lesdits épitopes d'anticorps sont dérivés d'un groupe d'antigènes comprenant des antigènes tumoraux, bactériens, parasitaires, viraux ou des auto-antigènes.

7. Protéine de fusion selon la revendication 6, où ledit ou lesdits épitopes d'anticorps sont dérivés d'antigènes de papillomavirus humain.

8. Protéine de fusion selon l'une quelconque des revendications 5-7 capable de donner naissance à une réponse anticorps protectrice.

9. Protéine de fusion selon la revendication 8 où ladite réponse anticorps protectrice est susceptible de réactions croisées à l'égard de deux ou plusieurs sous-types de papillomavirus humains définis du point de vue sérologique.

10. Protéine de fusion selon la revendication 9 où ladite réponse protectrice est produite contre deux ou plusieurs du groupe comprenant les protéines HPV-L1 dérivées de papillomavirus humain impliquées dans l'induction de tumeurs.

11. Protéine de fusion selon la revendication 10 où ladite réponse anticorps protectrice est capable de réactions croisées à l'égard de deux ou plusieurs du groupe des protéines HPV-L1 comprenant les protéines L1 de HPV-16, HPV-18, HPV-31 et HPV-45.

12. Protéine de fusion selon l'une quelconque des revendications 1-11 en combinaison avec une protéine de capside mineure L2 de papillomavirus humain (protéine HPV-L2).

13. Protéine de fusion selon la revendication 12 où ladite protéine HPV-L2 est fusionnée avec un ou plusieurs peptides supplémentaires.

14. Protéine de fusion selon la revendication 13 où ledit ou lesdits peptides supplémentaires sont choisis dans un groupe d'antigènes comprenant des antigènes tumoraux, bactériens, parasitaires, viraux et des auto-antigènes.

15. Vaccin comprenant comme ingrédient actif une protéine de fusion selon l'une quelconque des revendications 1-14.

16. Polynucléotide codant la protéine de fusion selon l'une quelconque des revendications 1-14.

17. Vaccin comprenant comme ingrédient actif un polynucléotide selon la revendication 16.

18. Protéine de fusion selon l'une quelconque des revendications 1-14 destinée à être utilisée comme médicament.

19. Utilisation d'une protéine de fusion selon l'une quelconque des revendications 1-14 pour la fabrication d'un vaccin pour prévenir ou traiter des infections virales, bactériennes ou parasitaires.

20. Utilisation selon la revendication 19 pour prévenir ou traiter une infection à papillomavirus humain.

21. Utilisation d'une protéine de fusion selon l'une quelconque des revendications 1-14 pour la fabrication d'un vaccin pour prévenir ou traiter le développement de conséquences bénignes ou malignes d'une infection à papillomavirus humain.

22. Utilisation selon la revendication 21 où ladite infection à papillomavirus humain est choisie dans le groupe comprenant les verrues et la papillomatose laryngée.

23. Utilisation d'une protéine de fusion selon l'une quelconque des revendications 1-14 pour la fabrication d'un vaccin pour prévenir ou traiter un cancer.

24. Utilisation selon la revendication 23 où ledit cancer est choisi dans le groupe comprenant le cancer du col, du pénis, de la vulve, du vagin, de l'anus et de l'oropharynx.
